# EUROPEAN PATENT SPECIFICATION

(11) **EP 0 467 422 B1**
(45) Date of publication and mention of the grant of the patent: **17.12.1997**
(21) Application number: 91116947.2
(22) Date of filing: 10.10.1983
(51) Int. Cl.: A61N 1/05, A61M 25/01

(54) **Guiding catheter**
Führungskatheter
Cathéter de guidage

(30) Priority: 14.10.1982 US 434318
(43) Date of publication of application: 22.01.1992
(62) Divisional of application: 83306120.3
(73) Proprietor: BAXTER INTERNATIONAL INC., Deerfield, IL 60015 (US)
(72) Inventor: Swendson, David Lyman, Garden Grove, CA 92645 (US); Elson, Edward Earl, Anaheim, California 92806 (US); Lieber, Clement Eugene, Yorba Linda, CA 92686 (US); Rold, Michael D., Cost Mesa, CA 92627 (US)
(74) Representative: Day, Jeremy John

(56) References cited:
- WO-A-81/03733
- DE-A- 2 605 590
- US-A- 3 169 528
- US-A- 3 995 623
- US-A- 4 332 259

## Description

This invention provides a guiding catheter by means of which a flexible pacing catheter can be introduced into the heart of the patient. DE-A-2605590 discloses a composite pacing catheter for pacing the right atrium and ventricle.

The invention provides a guiding catheter which readily forms into a gentle bend or curve in heart and does not kink when used with a pacing catheter. One reason that a composite catheter system tends to kink is that the catheter system proximally of the port out of which the pacing catheter emerges is relatively stiff, and the guiding catheter distally of the port is relatively flexible. This abrupt change in stiffness at the port tends to cause kinking of the guiding catheter.

With this invention, a stiffening element, such as an elongated polymeric or metallic wire, is permanently fixed, as by bonding, within the guiding catheter. The stiffening element begins proximally of the port and extends to a location distally of the port so that the region through the port and distally thereof is stiffened. The degree of stiffening is such as to permit the guiding catheter to form into the desired gentle curve without kinking. Preferably, the stiffening element terminates no farther distally than the right ventricle and no farther proximally than the superior vena cava. When the catheter system is used for pacing the right ventricle, the stiffening element preferably terminates at its opposite ends in the right atrium and the right ventricle.

The guiding catheter of the invention may for example, be used in conjunction with a pacing catheter as described in EP-A-0109178, from which this application is divided.

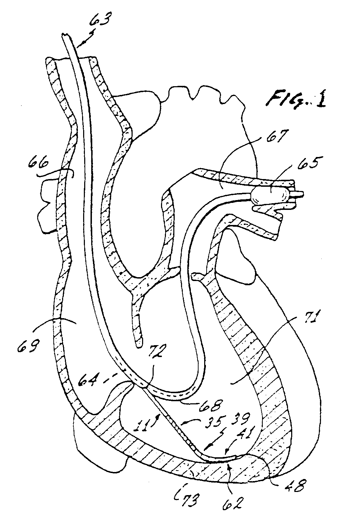

The invention, together with additional features and advantages thereof, may best be understood by reference to the following description taken in connection with the accompanying illustrative drawing, in which Fig.1 is a sectional view through a human heart showing one form of catheter according to the invention and the way in which it can be used.

Fig. 1. shows the guiding catheter of the invention.

Except for a stiffening element 64, the guiding catheter 63 may be of conventional construction and may be a Swan-Ganz thermodilution catheter which is available from American Edwards Laboratories of Irvine, California. The guiding catheter 63, which may have multiple lumens extending longitudinally through a catheter body, may be inserted into the heart through a vein using conventional techniques, and following such insertion, a balloon 65 adjacent the distal end of the guiding catheter is lodged in the pulmonary artery 67. As shown in Fig.1, the catheter 63 extends through the superior vena cava 66 and is formed into a curve 68 of about 180 degrees as it extends through the right atrium 69 and the right ventricle 71. The guiding catheter 63 has a port 72 leading from one of its lumens into the right ventricle 71.

In the embodiment illustrated, the stiffening element 64 is in the form of an elongated, flexible, resilient wire of metal or plastic bonded into the guiding catheter 63 outside of the lumen with which the port 72 communicates. In the preferred construction illustrated, the stiffening element 64 extends from a location in the right atrium 69 proximally of the port 72 continuously to a location in the right ventricle 71 located distally of the port 72. Thus, regions of the guiding catheter on the opposite sides of the ports 72 are stiffened, and such stiffening is controlled to cause the catheter 63 to form the relatively gentle curve 68 in the right heart without kinking as the catheter extends through the right heart to the pulmonary artery 67.

With the guiding catheter 63 positioned in the right heart as shown in Fig.1, a pacing catheter 11 of the type described in EP-A-0109178 can be inserted through a lumen of the guiding catheter 63 and out the port 72.

## Claims

1. An apparatus comprising :
a guiding catheter adapted to be passed through the right side of the heart into the pulmonary artery;
said guiding catheter including an elongated catheter body having proximal and distal ends, at least one lumen extending longitudinally in the body and a port extending from the lumen to the exterior of the catheter body, said port being in the right heart when the catheter extends through the right heart to the pulmonary artery;
said guiding catheter including an elongated stiffening element permanently fixed within the catheter body and extending from a location on the proximal side of said port to a location on the distal side of said port, said stiffening element being flexible but sufficiently stiff to cause the catheter to be gently curved without forming a kink when the catheter extends through the right ventricle to the pulmonary artery; and
said lumen being adapted to have an inner catheter passed through the lumen and the port into the right heart.

2. Apparatus according to claim 1 including an inner catheter extending through said lumen and passing through said port, said inner catheter including a pacing catheter having at least one electrode.

3. Apparatus according to claim 1 or claim 2, wherein said stiffening element extends no further proximally than the superior vena cava and no further distally than the right ventricle, when the guiding catheter extends through the right heart to the pulmonary artery.

4. Apparatus according to any one of claims 1 to 4, wherein the stiffening element is bonded into the catheter body.

## Patentansprüche

1. Vorrichtung, die folgendes umfaßt:
- einen Führungskatheter, der angepaßt ist, um durch die rechte Seite des Herzens hindurch in die Lungenarterie hineingeführt zu werden;
- der Führungskatheter, der einen verlängerten Katheterkörper mit proximalen und distalen Enden, wenigstens ein Lumen, das sich längs in den Körper erstreckt und einen Anschluß, der sich von dem Lumen zu dem Äußeren des Katheterkörpers erstreckt, umfaßt, wobei der Anschluß in dem rechten Herz ist, wenn der Katheter sich durch das rechte Herz zu der Lungenarterie erstreckt;
- der Führungskatheter, der ein verlängertes, versteifendes Element beinhaltet, das dauerhaft in dem Katheterkörper angebracht ist und sich von einem Ort auf der proximalen Seite des Anschlusses zu einem Ort auf der distalen Seite des Anschlusses erstreckt, wobei das versteifende Element flexibel aber ausreichend steif ist, um zu bewirken, daß der Katheter sanft gekrümmt wird, ohne einen Knick auszubilden, wenn der Katheter sich durch die rechte Herzkammer zu der Lungenarterie erstreckt; und
- das Lumen, das angepaßt ist, einen inneren Katheter zu haben, der durch das Lumen hindurchgeführt wird und das den Anschluß in das rechte Herz hinein hat.

2. Vorrichtung nach Anspruch 1,
die einen inneren Katheter umfaßt, der sich durch das Lumen erstreckt und durch den Anschluß hindurchführt, wobei der innere Katheter einen Schrittmacher-Katheter mit wenigstens einer Elektrode aufweist.

3. Vorrichtung nach Anspruch 1 oder 2,
wobei sich das versteifende Element nicht weiter proximal erstreckt als die Vena Cava Superior und es sich nicht weiter distal erstreckt als die rechte Herzkammer, wenn der Führungskatheter durch das rechte Herz hindurch zu der Lungenarterie erstreckt.

4. Vorrichtung nach einem der Ansprüche 1 bis 4,
wobei das versteifende Element in den Katheterkörper hineinverbunden ist.

## Revendications

1. Appareil comprenant :
un cathéter de guidage adapté pour être introduit dans la partie droite du coeur en direction de l'artère pulmonaire ;
ledit cathéter de guidage comprenant un corps de cathéter de forme allongée avec des extrémités distale et proximale, au moins une lumière qui s'étend longitudinalement dans le corps, et un orifice qui s'étend de la lumière vers l'extérieur du corps de cathéter, ledit orifice se trouvant dans la partie droite du coeur lorsque le cathéter passe dans la partie droite du coeur en direction de l'artère pulmonaire ;
ledit cathéter de guidage comprenant un élément raidisseur de forme allongée fixé de manière permanente à l'intérieur du corps de cathéter et s'étendant d'un emplacement sur le côté proximal dudit orifice jusqu'à un emplacement sur le côté distal dudit orifice, ledit élément raidisseur étant flexible mais suffisamment rigide pour amener le cathéter à prendre une forme légèrement incurvée sans plicature lorsque le cathéter passe dans le ventricule droit en direction de l'artère pulmonaire ; et
ladite lumière étant adaptée pour pouvoir faire passer un cathéter interne par ladite lumière et ledit orifice dans la partie droite du coeur.

2. Appareil selon la revendication 1, comprenant un cathéter interne qui s'étend dans ladite lumière et passe par ledit orifice, ledit cathéter interne comprenant un cathéter de stimulation qui possède au moins une électrode.

3. Appareil selon la revendication 1 ou la revendication 2, dans lequel ledit élément raidisseur ne s'étend pas dans une position plus proximale que la veine cave supérieure ni dans une position plus distale que le ventricule droit lorsque le cathéter de guidage passe dans la partie droite du coeur en direction de l'artère pulmonaire.

4. Appareil selon l'une quelconque des revendications 1 à 4, dans lequel l'élément raidisseur est collé dans le corps du cathéter.
